Europäisches Patentamt

European Patent Office

Office européen des brevets

(11) Numéro de publication: **0 238 769 B1**

(12) **FASCICULE DE BREVET EUROPEEN**

(45) Date de publication de fascicule du brevet:
**31.07.91**

(51) Int. Cl.⁵: **G01N 33/18**

(21) Numéro de dépôt: **86402928.5**

(22) Date de dépôt: **24.12.86**

(54) **Procédé d'étalonnage d'un automate de dosage de composés hydrocarbonés en milieu aqueux.**

(30) Priorité: **24.12.85 FR 8519129**

(43) Date de publication de la demande:
**30.09.87 Bulletin 87/40**

(45) Mention de la délivrance du brevet:
**31.07.91 Bulletin 91/31**

(84) Etats contractants désignés:
**BE DE ES GB IT NL**

(56) Documents cités:
**GB-A- 1 259 691**

**DERWENT JAPANESE PATENTS GAZETTE, section CH: chemical, semaine B13, 10 mai 1979, Chemical Engineering, Derwent Publications LTD, Londres (GB); p. 8**

(73) Titulaire: **COMPAGNIE GENERALE DES EAUX 52 rue d'Anjou F-75008 Paris(FR)BE DE ES GB IT NL**

Titulaire: **SYNDICAT DES EAUX D'ILE DE FRANCE Etablissement public dit: Tour de Lyon 185 rue de Bercy F-75012 Paris(FR)BE DE ES GB IT NL**

(72) Inventeur: **Gibert, Michel 48 rue des Martyrs F-75009 Paris(FR)**

(74) Mandataire: **Cuer, André et al Cabinet Ballot-Schmit 7, rue Le Sueur F-75116 Paris(FR)**

EP 0 238 769 B1

## Description

La présente invention a trait au domaine de l'analyse des composés hydrocarbonés contenus dans un milieu aqueux, comme par exemple la recherche et la détermination des hydrocarbures totaux renfermés dans une eau polluée. Elle a trait tout particulièrement à un procédé d'étalonnage d'un analyseur automatique ou automate destiné à doser en continu les quantités d'agent hydrocarboné dans l'eau.

Lorsqu'il s'agit d'analyser au laboratoire ou de façon non périodique un taux de composé hydrocarboné (appelé ci-après hydrocarbure, dans un but de simplification) dans l'eau, on peut se contenter d'étalonner le dispositif de mesure à partir de solutions d'hydrocarbures dans un solvant non aqueux choisi, transparent aux rayons infrarouges du fait que la majorité des mesures s'effectue à l'aide de spectrophotomètres I.R.

Pour entreprendre des analyses en continu et de façon systématique, il faut disposer d'un automate dont la solution étalon doit alors subir les différentes étapes du processus de mesure dont : efficacité des étapes de rinçage pour assurer la propreté rigoureuse du circuit, bon fonctionnement de l'extraction, détection fiable. Dans les automates qui existent à l'heure actuelle, l'étalonnage, au moyen d'une réserve de solution étalon, s'effectue lors de la phase précitée de détection. Il n'est alors pratiquement pas possible de vérifier que l'ensemble du système présente un fonctionnement convenable et sûr.

Certes, un moyen idéal de constituer un étalon pour un automate du type précité serait de disposer d'une eau contenant une quantité extrêmement faible d'hydrocarbure. Toutefois, ceci est difficilement envisageable du fait des difficultés d'introduire une quantité connue d'hydrocarbures de façon homogène dans de l'eau.

L'invention permet de pallier les difficultés susmentionnées et de résoudre le problème de l'étalonnage d'un automate par une solution ou dispersion d'hydrocarbure dans l'eau, facile à préparer et à conserver et permettant d'obtenir des résultats fiables et répétitifs.

Conformément à l'invention, on utilise comme étalon une solution aqueuse contenant $10^{-5}$ à $10^{-3}$ g/l d'eau d'un agent tensioactif dont la chaine hydrocarbonée comporte au moins 8 atomes de carbone et dont l'équivalent en hydrocarbure donné est connu.

Le choix de l'agent tensioactif, selon la définition générale ci-dessus, est fonction d'un certain nombre de critères :

- la chaine hydrocarbonée du tensioactif doit être suffisamment longue pour donner un signal, par exemple aux I.R., similaire à celui des hydrocarbures habituellement rencontrés comme impuretés de l'eau (lorsqu'il s'agit d'analyse d'eaux polluées). A cet égard, la présence de 8 atomes de carbone, en chaine linéaire ou ramifiée, constitue un minimum, la condensation en carbone pouvant aller jusqu'à 20 ou plus ;
- la balance ou équilibre hydrophile-hydrophobe (ou lipophile) doit permettre à la fois l'obtention d'une solution ou dispersion aqueuse stable mais aussi un rendement correct d'extraction par le solvant organique choisi ;
- le produit étalon doit présenter une bonne stabilité à l'hydrolyse et/ou à la biodégrabilité dans les conditions de conservation, encore que, pour ce dernier point, on puisse ajouter à la solution étalon des bactéricides.

Parmi les agents tensioactifs aptes à satisfaire à l'ensemble de ces caractéristiques, dans le cadre de l'invention, on peut citer à titre préférentiel :

. des agents anioniques dont l'anion correspond à des acides faibles. A cet égard, il est préférable d'éliminer les anions des types : sulfate, sulfonate, phosphate... et de retenir, comme spécialement avantageux, des acides gras, naturels ou synthétiques, saturés ou non, comme par exemple ceux de condensation en carbone C12 à C18 ;

. des tensioactifs non ioniques qui peuvent comprendre des produits hydrocarbonés à fonction alcool, éther, amine, amide... etc. Parmi ceux-ci on peut citer à titre indicatif des condensats d'oxyde d'éthylène et d'alcoylphénols obtenus à partir de 4 à 8 molécules d'oxyde d'éthylène.

La dose optimum de l'agent tensioactif est choisie en fonction de sa réponse pour donner un signal de même importance que les hydrocarbures dans la gamme de concentration prévue. En pratique, la quantité d'agent tensioactif introduit dans la solution ou dispersion aqueuse servant comme étalon est généralement comprise entre $10^{-5}$ et $10^{-3}$ grammes par litre d'eau, par exemple 50 microgrammes à 1 mg/l. La bonne solubilité de dispersion de l'agent tensioactif dans l'eau permet d'atteindre ces concentrations par dilution de solutions mères. Les prises d'essai pour analyse, par exemple dans le cas d'eau polluée, sont habituellement de l'ordre de 500 ml à 1 litre.

La correspondance entre la réponse de l'agent tensio-actif choisi et celle de l'hydrocarbure étalon peut être faite une fois pour toute au laboratoire. Il est ainsi possible de préparer des solutions étalons dont l'équivalent hydrocarbure est connu.

Comme déjà signalé, un automate étalonné selon l'invention permet de déterminer l'ensemble des composés hydrocarbonés, exprimés en hydrocarbures totaux, contenus à titre d'impuretés dans un milieu aqueux, comme par exemple une eau polluée à purifier. Parmi ces impuretés hydrocarbonnées les plus couramment rencontrées, on peut citer par exemple, à titre non limitatif, l'essence, le kérosène, des fuels légers ou lourds...etc.

A titre d'exemples de réalisation non limitatifs, conformes à l'invention, on signalera ci-dessous la confection d'étalons à partir d'une part de palmitate de sodium (tiré de l'acide linéaire en C16) et, d'autre part, de condensat de nonylphénol avec 6 molécules d'oxyde d'éthylène (produit dénommé ici NP6).

Pour chacun des deux produits, on a préparé une gamme étalon dans le solvant d'extraction choisi pour les hydrocarbures (par exemple tétrachlorure de carbone ou trichlorotrifluoréthane). Les mesures directes des signaux en infra-rouge dans la zone d'absorption des fonctions hydrocarbonées (3000 cm$^{-1}$) ont permis d'établir les droites d'étalonnage pour ces produits dans la gamme des concentrations désirées. Le rapport des pentes de ces droites avec les pentes des hydrocarbures à mesurer dans le même solvant donnait le coefficient de correction à appliquer pour l'étalonnage avec les tensioactifs. On a alors préparé des solutions mères d'agent tensioactif à 100 mg/l d'eau que l'on a diluées jusqu'à obtention de concentrations comprises entre 0,05 et 0,5 mg/l d'eau. Puis on a appliqué aux solutions préparées la procédure d'extraction et de mesure retenue pour les hydrocarbures. Les teneurs mesurées à partir des droites étalon obtenues avec le solvant d'extraction étaient alors comparées aux teneurs initiales afin d'établir le rendement d'extraction.

En opérant ainsi on a pu obtenir des rendements de 91 % pour le NP$_6$ et de 98 % pour le palmitate de sodium. De tels rendements sont compatibles avec la précision souhaitable pour la détermination de traces de composés hydrocarbonés. En outre, on a constaté qu'après conservation des solutions étalons à température ambiante pendant diverses périodes (au moins 1 semaine) les résultats obtenus étaient similaires.

## Revendications

1. Procédé d'étalonnage d'un automate destiné à mesurer la quantité d'hydrocarbures totaux contenus dans un milieu aqueux à partir de solutions-étalons dont l'équivalent en composé hydrocarboné donné est connu, caractérisé en ce que l'on utilise comme étalon une solution aqueuse contenant 10$^{-5}$ à 10$^{-3}$ g/l d'eau d'un agent tensioactif dont la chaine hydrocarbonée comporte au moins 8 atomes de carbone.

2. Procédé selon la revendication 1, caractérisé en ce que l'agent tensioactif est choisi dans le groupe des agents anioniques dont les anions correspondent à des acides faibles tels que des acides gras naturels ou synthétiques, saturés ou non, de condensation en carbone 8 à 20 ou plus.

3. Procédé selon la revendication 1, caractérisé en ce que l'agent tensioactif est choisi dans le groupe des agents non ioniques tels que alcools, éthers, amines, amides, alcoylphéncls, condensats d'oxyde d'éthylène et de produits à OH libres; lesdits agents ayant une condensation en carbone de 8 à 20 ou plus.

4. Application du procédé selon l'une quelconque des revendications 1 à 4 à l'analyse automatique d'une eau polluée par des composés hydrocarbonés.

## Claims

1. Process for calibrating an automaton for measure of whole hydrocarbons quantity contained in an aqueous medium from standard solutions of which the equivalent in given hydrocarbon compound is known, characterized in that is used as standard an aqueous solution containing 10$^{-5}$ to 10$^{-3}$ g by water liter of a tensioactiv agent of which the hydrocarbon radical comprises at least 8 carbon atoms.

2. Process according the claim 1, wherein the hydrocarbon agent is selected from the group consisting off anionic agents of which the anions correspond to weak acids, such as natural or synthetic fatty acids, saturated or not, of carbon condensation of 8 to 20 or more.

3. Process according the claim 1, wherein the tensioactiv agent is selected from the group consisting off unionic agents such as alcohols, ethers, amins, amids, alkylphenols, condensates of ethylen oxide and products with free OH ; the said agents comprising 8 to 20 or more carbon atoms.

4. Use of the process according anyone of claims 1 to 3, for the automatic analysis of a polluted water by hydrocarbon compounds.

## Patentansprüche

1. Verfahren zur Elchung eines Automaten, der zur Feststellung der Menge der in einem wässrigen Medium enthaltenen Kohienwasserstoffe unter Anwendung von Eichlösungen bestimmt ist, bei denen der Aquivalentwert der jeweiligen Kohlenwasserstoffverbindung bekannt ist, gekennzeichnet durch die Anwendung einer wässrigen Eichlösung mit $10^{-5}$ bis $10^{-3}$ g eines Tensidwirkstoffs, deren Kohlenwasserstoffkette mindestehs 8 Kohlenstoffatome enthält, je liter Wasser

2. Verfahren nach Anspruch 1, dadurch gekennzeichnet, daß der Tensidwirkstoff aus der Gruppe der anionischen Wirkstoffe gewählt wird, deren Anionen schwachen Säuren wie z. B. natürlichen oder synthetlechen, gesättigten oder ungesättigten Fatteäuren mit 8 bis 20 oder mehr Kohlenstoffatomen in dar Kette entsprechen.

3. Verfahren nach Anspruch 1, dadurch gekennzeichnet, daß der Tensidwirkstoff aus der Gruppe der nichtionlschen Wirkstoffen wie z. B. Alkoholen, Athern Aminen, Alkylphenden, Äthylenoxidkondensaten und frelen OH-Produkte ausgewählt ist, wobei die Wirkstoffe 8 bis 20 oder mehr Kohlenstoffatome in der kette enthalten.

4. Verwendung des Verfahrens nach einem der Ansprüche 1 bis 4 für die automaisierte Analyse von mit Kohlenwasserstoffen verunreinigtem Wasser.